# EUROPEAN PATENT APPLICATION

(11) **EP 4 382 908 A1**
(43) Date of publication of application: **12.06.2024**
(21) Application number: 22853143.0
(22) Date of filing: 04.08.2022
(51) Int. Cl.: G01N 33/53, C07K 16/18, G01N 33/531

(54) **IMMUNOASSAY FOR THYROGLOBULIN AND KIT THEREFOR**

(30) Priority: 06.08.2021 JP 2021130019
(71) Applicant: Advanced Life Science Institute, Inc., Tokyo 192-0031 (JP)
(72) Inventor: ARAI, Osamu, Hachioji-shi, Tokyo 192-0031 (JP); YAGI, Shintaro, Hachioji-shi, Tokyo 192-0031 (JP); AOYAGI, Katsumi, Hachioji-shi, Tokyo 192-0031 (JP)
(74) Representative: Mewburn Ellis LLP
(86) International application number: PCT/JP2022/029921
(87) International publication number: WO 2023/013725

(57) **Abstract**

Provided is a novel method that enables accurate measurement of thyroglobulin content in a sample even when the sample is positive for an anti-thyroglobulin antibody. The method is a method of measuring thyroglobulin by immunoassay in a sample isolated from the body, including a pretreatment step of treating the sample isolated from the body with a reducing agent, wherein a monoclonal antibody or an antigen-binding fragment thereof whose corresponding antigen is thyroglobulin treated with the reducing agent is used for the immunoassay.

## Description

### TECHNICAL FIELD

The present invention relates to an immunoassay of thyroglobulin and a kit therefor.

### BACKGROUND ART

Thyroglobulin (Tg) is a glycoprotein having a molecular weight of 660,000 produced only in thyroid follicular cells. Biosynthesized Tg is released into the follicular lumen. In this process, binding of iodine molecules to tyrosine groups in the Tg molecule occurs by the action of peroxidase, to cause synthesis of thyroid hormone. Tg in the follicular lumen is incorporated again into the follicular cells, and decomposed in the follicular cells to cause release of the thyroid hormone. This process is activated by the action of thyroid-stimulating hormone (TSH). Thus, under normal conditions, release of Tg itself into blood hardly occurs, and release of Tg into blood indicates a certain abnormality of the thyroid. Therefore, Tg is an extremely useful marker for thyroid diseases because of its high organ specificity. In particular, blood Tg is used as a marker for evaluation of operations for differentiated thyroid cancer, and as a marker for knowing the presence or absence of postoperative recurrence or metastasis. In addition, for example, it is useful as an index of the effect of treatment and remission of Basedow disease, and also useful for identification or differential diagnosis of the disease type, and therapeutic monitoring, of congenital hypothyroidism. It has also been suggested that combination with diagnostic imaging may enable preoperative diagnosis of nodular goiter and differential diagnosis between a benign thyroid disease and a malignant tumor.

However, in cases where the subject is positive for anti-thyroglobulin antibody (TgAb), a low Tg value may be found due to a problem in the measurement even when the actual value of Tg is high. For example, since 20 to 30% of patients with thyroid cancer are positive for TgAb, simultaneous measurement of TgAb has been necessary for measurement of Tg. Further, accurate measurement of the amount of Tg is difficult in cases of Hashimoto disease with TgAb positivity, and, similarly, there has been a possibility that the amount of Tg measured in cases of another autoimmune disease showing TgAb positivity (Basedow disease) has been inaccurate.

A known method of thyroglobulin measurement involves pretreating a sample with a surfactant or an acidifier in order to accurately measure the Tg content (Patent Document 1). Patent Document 1 suggests that a pretreatment solution may include a reducing agent as an optional ingredient, but such a pretreatment solution is not used in the examples. In addition, another known method of thyroglobulin measurement involves pretreating a sample with an alkaline substance (Patent Document 2). Furthermore, another known method of thyroglobulin measurement method uses an anti-Tg monoclonal antibody generated using a synthetic peptide as an immunogen (Patent Document 3).

### PRIOR ART DOCUMENT

### Patent Documents

Patent Document 1: WO 2018/047792
Patent Document 2: WO 2020/0241443
Patent Document 3: JP H7-46966 A

### SUMMARY OF THE INVENTION

### PROBLEMS TO BE SOLVED BY THE INVENTION

An object of the present invention is to provide a novel method that enables accurate measurement of thyroglobulin content in a sample even when the sample is positive for an anti-thyroglobulin antibody.

### MEANS FOR SOLVING THE PROBLEMS

The present inventors have conducted extensive research and consequently found that the thyroglobulin/anti-thyroglobulin antibody immune complex can be dissociated by reduction treatment, and have succeeded in producing a monoclonal antibody whose corresponding antigen is thyroglobulin dissociated by treatment with a reducing agent, and have found that an immunoassay using the monoclonal antibody allows accurate measurement of thyroglobulin even in a sample that is positive for an anti-thyroglobulin antibody, to complete the present invention.

That is, the present invention provides the following:
(1) A method of measuring thyroglobulin by immunoassay in a sample isolated from the body, comprising a pretreatment step of treating the sample isolated from the body with a reducing agent, wherein a monoclonal antibody or an antigen-binding fragment thereof whose corresponding antigen is thyroglobulin treated with the reducing agent is used for the immunoassay.
(2) The method of (1), wherein the corresponding epitope of the monoclonal antibody is contained in an amino acid sequence of any one of SEQ ID NOs: 1 to 5.
(3) The method of (1) or (2), wherein the immunoassay is a sandwich assay and wherein the monoclonal antibody or an antigen-binding fragment thereof is used as at least one of a first antibody or a second antibody in the sandwich assay.
(4) The method of (3), wherein the first antibody comprises at least one selected from the group consisting of an antibody whose corresponding epitope is contained in the amino acid sequence of SEQ ID NO: 1 and an antibody whose corresponding epitope is contained in the amino acid sequence of SEQ ID NO: 5, and wherein the second antibody comprises at least one selected from the group consisting of an antibody whose corresponding epitope is contained in the amino acid sequence of SEQ ID NO: 2, an antibody whose corresponding epitope is contained in the amino acid sequence of SEQ ID NO: 3, and an antibody whose corresponding epitope is contained in the amino acid sequence of SEQ ID NO: 4.
(5) The method of (4), wherein the first antibody is a capture antibody and the second antibody is a labeled antibody.
(6) An immunoassay kit for use in the method of (1), comprising a reducing agent and a monoclonal antibody or an antigen-binding fragment thereof whose corresponding antigen is thyroglobulin treated with the reducing agent.

### EFFECT OF THE INVENTION

The present invention allows accurate measurement of thyroglobulin even in a sample that is positive for an anti-thyroglobulin antibody.

### BRIEF DESCRIPTION OF THE DRAWINGS

Fig. 1A shows the epitope analysis results obtained in the examples described below.
Fig. 1B shows the same.
Fig. 1C shows the same.
Fig. 2 shows the effect of pretreatment with different types of reducing agents obtained in the examples described below.
Fig. 3 shows the correlation between a Tg-SH measurement system and a Tg measurement system obtained in the examples described below.
Fig. 4 shows the correlation between a Tg-SH measurement system and a Tg measurement system in a low concentration range obtained in the examples described below.

### MODE FOR CARRYING OUT THE INVENTION

Unless otherwise specified, each "%"-based concentration described in the present description represents a weight/volume (w/v)-based concentration. In cases where the temperature is not specified, the temperature is room temperature.

### <Method of Measuring thyroglobulin>

The thyroglobulin (Tg) to be measured in the present invention is Tg derived from an arbitrary animal. The Tg is preferably Tg derived from a mammal (for example, a primate such as human, monkey, or chimpanzee; a rodent such as mouse, rat, or rabbit; a pet animal such as dog or cat; a domestic animal such as pig or cow; or a working animal such as horse or sheep), more preferably Tg derived from a primate, especially preferably Tg derived from human.

### 1. Pretreatment Step

The method of the present invention is a method in which Tg in a sample is measured by an immune reaction in which the sample is reacted with an antibody, and includes a pretreatment step in which the sample is mixed with a pretreatment solution prior to the immune reaction (reaction step). The pretreatment step can retain Tg in a state released from the autoantibody (TgAb) or the like. The pretreatment solution contains a reducing agent. The reducing agent used may be any existing reducing agent such as 2-(diethylamino)ethanethiol hydrochloride (2-DEAET), dithiothreitol (DTT), tris(2-carboxyethyl)phosphine hydrochloride (TCEP), 2-mercaptoethanol, mercaptoethylamine, or cysteine. 2-DEAET, TCEP, and DTT are particularly suitable for use. As the concentration of the reducing agent, a concentration is preferred at which the reducing agent can dissociate the Tg/anti-Tg antibody immune complex to remove the influence of TgAb. This concentration varies depending on the reducing agent used and can be explored by the method specifically explained in the examples described below. Briefly, a sample is pretreated with a reducing agent at various concentrations and the resulting sample is subjected to an immunoassay under identical conditions except for the presence or absence of TgAb. The preferred concentration is a concentration at which the immunoassay measures the same level of values in the presence and absence of TgAb. As specifically explained in the examples described below, the preferred concentration (a final concentration of a reducing agent after mixing with a sample in the pretreatment) is about 150 mM or more for 2-DEAET, about 10 mM or more for DTT, and about 5 mM or more for TCEP. The upper limit of the concentration of a reducing agent is not specifically limited. However, increasing the concentration of a reducing agent is pointless if the increased concentration of the reducing agent has no further effect on TgAb. Therefore, the preferred concentration is typically not more than 4 times the concentrations described above and preferably not more than twice the concentrations described above. That is, the upper limit of the concentration of 2-DEACT is, for example, 600 mM and preferably 300 mM; the upper limit of the concentration of DTT is, for example, 40 mM and preferably 20 mM; the upper limit of the concentration of TCEP is, for example, 20 mM and preferably 10 mM.

A sample used in the present invention is not specifically limited as long as the sample can contain Tg. Examples of the sample include serum, plasma, whole blood, urine, feces, oral mucosa, pharyngeal mucosa, intestinal mucosa, and biopsy specimens (for example, thyroid fine needle aspiration (FNA) specimen, intestinal specimen, liver specimen). Preferably, the sample is a blood sample (whole blood, serum, or plasma). More preferably the sample is serum or plasma.

The pretreatment solution may contain another protein denaturant, such as urea or thiourea, if necessary. The concentration of the denaturant, in terms of the concentration during the treatment, is preferably not less than 0.1 M, more preferably not less than 0.5 M and less than 4 M. For enhancement of the effect of the treatment, the pretreatment liquid may contain any of monosaccharides, disaccharides, citric acid, and citric acid salts, or a combination of these. The pretreatment liquid may also contain a chelating agent such as EDTA. The pretreatment solution may also contain a surfactant. Any of anionic surfactants, cationic surfactants, zwitterionic surfactants, and nonionic surfactants can be used as the surfactant. Examples of the zwitterionic surfactant include C10APS (N-decyl-*N,N*-dimethyl-3-ammonio-1-propanesulfonate), C12APS (*N*-dodecyl-*N*,*N*-dimethyl-3-ammonio-1-propanesulfonate), C14APS (*N*-tetradecyl-*N*,*N*-dimethyl-3-ammonio-1-propanesulfonate), and C16APS (*N*-hexadecyl-*N,N*-dimethyl-3-ammonio-1-propanesulfonate). Examples of the anionic surfactant include sodium dodecyl sulfate (SDS), *N*-lauroylsarcosine (NLS), lithium dodecyl sulfate, sodium dodecyl benzenesulfonate, and deoxycholic acid. Examples of the cationic surfactant include decyltrimethylammonium chloride, dodecyltrimethylammonium chloride, tetradecyltrimethylammonium chloride, hexadecyltrimethylammonium chloride (C16TAC), decyltrimethylammonium bromide, dodecyltrimethylammonium bromide, tetradecyltrimethylammonium bromide, hexadecyltrimethylammonium bromide (CTAB), laurylpyridinium chloride, tetradecylpyridinium chloride, and cetylpyridinium chloride. Examples of the nonionic surfactant include polyoxyethylene isooctylphenyl ethers, such as Triton X100 and Triton X114; polyoxyethylene nonylphenyl ethers, such as NP-40; and polyoxyethylene sorbitan alkyl esters, such as Tween 80. The surfactant content is, for example, from 1 to 10%, preferably from 3 to 8%, based on the weight of the pretreatment solution.

The temperature of the pretreatment step is not specifically limited as long as the temperature does not adversely affect the immunoassay. For example, the pretreatment can be performed at a temperature of 0°C to 37°C and can be performed at room temperature without any problems. Therefore, it is preferable to perform the pretreatment at room temperature for convenience.

### 2. Reaction Step

The sample mixture obtained in the above-described pretreatment step of the method of the present invention is then subjected to the reaction step of the immunoassay. In the reaction step, the sample mixture is optionally mixed with a buffer and the antigen in the mixture is reacted with an antibody against Tg or an antigen-binding fragment thereof. Regarding the immunoassay itself of Tg, a variety of methods are well known, and any immunoassay capable of quantification of Tg may be employed.

Examples of the buffer include those based on MES buffer, phosphate buffer, Tris buffer, and carbonate buffer. Buffers based on phosphate buffer may be especially preferably used.

The antibody used in the method of the present invention is a monoclonal antibody whose corresponding antigen is Tg treated with a reducing agent (hereinafter sometimes referred to as "Tg-SH"). As used herein, the phrase "whose corresponding antigen is Tg-SH" refers to a monoclonal antibody that is raised against Tg-SH as an immunogen and undergoes an antigen-antibody reaction with Tg-SH. A monoclonal antibody whose corresponding antigen is Tg-SH can be obtained by the conventional hybridoma technique (see the examples described below) using Tg-SH as the immunogen. The monoclonal antibody is not restricted to a specific isotype, but can be any of IgG, IgM, IgA, IgD, IgE, and IgY. In addition, an antigen-binding fragment of the above monoclonal antibody, such as F(ab')₂, Fab', Fab, or Fv, may also be used (in the following description (above the "EXAMPLES" section), the term "antibody" refers to "antibody or an antigen-binding fragment thereof' unless the context clearly indicates otherwise). In cases where two antibodies are used, as in a sandwich assay, at least one of the antibodies should be a monoclonal antibody whose corresponding antigen is Tg-SH, and the other may be a polyclonal antibody. However, it is preferable that both antibodies are monoclonal antibodies whose corresponding antigen is Tg-SH, as this will increase the reproducibility of the measured values.

In the examples described below, five monoclonal antibodies were obtained by the hybridoma technique using Tg-SH as the immunogen. The epitopes of the monoclonal antibodies are contained in the sequences of SEQ ID NOs: 1 to 5, respectively. The fact that five different monoclonal antibodies were obtained by the hybridoma technique using Tg-SH as the immunogen has indicated that a monoclonal antibody whose corresponding antigen is Tg-SH can be reproducibly obtained by the conventional hybridoma technique using Tg-SH as the immunogen.

The antibody against Tg-SH may be immobilized on a solid phase. In this specification, an antibody immobilized on a solid phase or to be immobilized on a solid phase may be referred to simply as an immobilized antibody. Examples of the solid phase include solid phases in which a liquid phase can be stored or loaded (for example, supports such as plates, membranes, and test tubes; and containers such as well plates, microchannels, glass capillaries, nanopillars, and monolith columns) and solid phases that can be suspended or dispersed in a liquid phase (for example, solid-phase carriers such as particles). Examples of the material of the solid phase include glasses, plastics, metals, and carbons. As the material of the solid phase, a non-magnetic material or a magnetic material may be used. From the viewpoint of simplicity of operation and the like, the material is preferably a magnetic material. The solid phase is preferably a solid-phase carrier, more preferably a magnetic solid-phase carrier, still more preferably a magnetic particle. As the method for immobilization of the antibody, a conventionally known method may be used. Examples of such a method include physical adsorption, covalent bonding, use of an affinity substance (biotin, streptavidin, or the like), and ionic bonding. In a particular embodiment, the antibody against Tg-SH is an antibody immobilized on a solid phase, preferably an antibody immobilized on a magnetic solid phase, and more preferably an antibody immobilized on a magnetic particle.

In the reaction step, after the mixing of the mixed liquid of the pretreatment step with the buffer, the resulting mixture may be brought into contact with the immobilized antibody, or, for example, an antibody immobilized on particles may be preliminarily included in a buffer to provide a particle liquid followed by mixing the above mixed liquid with the particle liquid. Although the reaction step may be carried out by a primary reaction step alone as in the immunoagglutination method or the competitive method, a secondary reaction step may also be provided as in the sandwich method. In cases where the secondary reaction step is provided, a washing step for removal of unreacted components may be provided between the primary reaction step and the secondary reaction step.

The antibody against Tg-SH may be labeled with a labeling substance. In the present description, an antibody labeled with a labeling substance may be simply referred to as a labeled antibody. Examples of the labeling substance include enzymes (for example, peroxidase, alkaline phosphatase, luciferase, β-galactosidase), affinity materials (for example, streptavidin, biotin), fluorescent substances or proteins (for example, fluorescein, fluorescein isothiocyanate, rhodamine, green fluorescent protein, red fluorescent protein), luminescent or light-absorbing substance (for example, luciferin, aequorin, acridinium, ruthenium), radioactive materials (for example, ³H, ¹⁴C, ³²P, ³⁵S, ¹²⁵I). In cases where a secondary reaction is included in the method of the present invention, an antibody used in the secondary reaction may be labeled with a labeling substance as described above.

In a particular embodiment, another antibody against Tg-SH which recognizes an epitope different from that recognized by the antibody against Tg-SH is included as an antibody used in a secondary reaction in the method of the present invention. The epitopes recognized by an antibody against Tg-SH and another antibody against Tg-SH are not limited to a specific combination. The use of another antibody as described above is preferred, for example, when using a sandwich assay. As described above, one of the antibodies may be a polyclonal antibody that undergoes an antigen-antibody reaction with Tg-SH.

### 3. Detection Step

In cases where a label is used for a primary antibody or a secondary antibody, the label is detected by a method relevant to the label used: for example, in cases where an enzyme is used as a label, the label is detected by adding a substrate of the enzyme. For example, in cases where alkaline phosphatase (ALP) is used for a labeled antibody, 3-(2'-spiroadamantane)-4-methoxy-4-(3'-phosphoryloxy)phenyl-1,2-dioxetane disodium salt (AMPPD) can be used as a substrate for the enzyme in a chemiluminescent enzyme immunoassay (CLEIA) system.

The method of the present invention is an immunoassay using an antibody against Tg-SH. Examples of such an immunoassay include the direct competitive method, indirect competitive method, and sandwich method. These types of immunoassays include chemiluminescent enzyme immunoassay (CLEIA), chemiluminescent immunoassay (CLIA), turbidimetric immunoassay (TIA), enzyme immunoassay (EIA) (for example, direct competitive ELISA, indirect competitive ELISA, and sandwich ELISA), radioimmunoassay (RIA), latex agglutination method, fluorescence immunoassay (FIA), and immunochromatography. These immunoassays *per se* are well known and do not need to be described in detail here, but each is briefly described below.

The direct competitive method is a method in which an antibody against a target antigen to be measured (in the present invention, Tg-SH) is immobilized on a solid phase (the solid phase and the immobilization are as described above), and blocking treatment (treatment of the solid phase with a solution of protein such as serum albumin) for prevention of non-specific adsorption is carried out, followed by reacting this antibody with a test sample containing the target antigen (in the present invention, a biological sample subjected to the pretreatment step as described above) and a certain amount of labeled antigen (the label is as described above), performing washing, and then quantifying the label bound to the solid phase. Since the antigen in the test sample and the labeled antigen competitively bind to the antibody, the larger the amount of the antigen in the test sample, the smaller the amount of the label bound to the solid phase. Antigen standard solutions with various known concentrations are prepared, and the amount of the label (the absorbance, luminescence intensity, fluorescence intensity, or the like depending on the properties of the label; the same applies hereinafter) immobilized on the solid phase is measured for each solution, followed by preparation of a calibration curve in which the antigen concentration is taken along the abscissa, and the amount of the label is taken along the ordinate. By measuring the amount of the label for an unknown test sample, and applying the measured amount of the label to the calibration curve, the amount of the antigen in the unknown test sample can be measured. The direct competitive method *per se* is well known in the art, and described in, for example, US 20150166678 A1.

In the indirect competitive method, a target antigen (in the present invention, Tg-SH) is immobilized on a solid phase (the solid phase and the immobilization are as described above). Subsequently, blocking treatment of the solid phase is carried out, and then a test sample containing the target antigen (in the present invention, a biological sample subjected to the pretreatment step as described above) is mixed with a certain amount of an anti-target-antigen antibody, followed by reaction with the immobilized antigen. After washing, the anti-target-antigen antibody bound to the solid phase is quantified. This can be carried out by allowing reaction with a labeled secondary antibody (the label is as described above) against the anti-target-antigen antibody, performing washing, and then measuring the amount of the label. Antigen standard solutions with various known concentrations are prepared, and the amount of the label immobilized on the solid phase is measured for each solution, followed by preparation of a calibration curve. By measuring the amount of the label for an unknown test sample, and applying the measured amount of the label to the calibration curve, the amount of the antigen in the unknown test sample can be measured. It is also possible to use a labeled primary antibody without using the labeled secondary antibody. The indirect competitive method *per se* is well known in the art, and described in, for example, the above-mentioned US 20150166678 A1.

Among sandwich assays, a forward sandwich assay, for example, is a method in which an anti-target antigen antibody is immobilized on a solid phase (the solid phase and the immobilization are as described above), optionally blocked, reacted with a test sample containing the target antigen (which in the present invention is a sample obtained by the pretreatment step described above), washed, reacted with a labeled secondary antibody (the label is as described above) against the target antigen, followed by washing and quantification of the label bound to the solid phase. For example, among sandwich assays, a reverse sandwich assay is a method in which a labeled antibody (label) against a target antigen is first reacted with a test sample, and an antigen-antibody complex resulting from the binding between the labeled antibody and the target antigen is then reacted with an immobilized antibody, followed by washing and quantification of the label bound to the solid phase. In addition, a sandwich assay is available in which an immobilized antibody, a test sample, and a labeled antibody are reacted simultaneously. In a sandwich assay, an antibody immobilized on a solid phase after reaction with a test sample can be used as an immobilized antibody. For example, when using an antibody immobilized on a solid phase, an antibody to be immobilized can be reacted with a test sample and a labeled antibody, followed by immobilization of the antibody immobilized on the solid phase, or an antibody to be immobilized can be reacted with a test sample, followed by immobilization of the immobilized antibody on the solid phase and reaction with a labeled antibody. Antigen standard solutions of various known concentrations are prepared and applied to the solid phase, and the amount of label is measured for each standard solution to generate a calibration curve. The amount of antigen in an unknown test sample can be determined by measuring the amount of label in the unknown test sample and interpolating the measured amount of label to the calibration curve. The sandwich assays are well known *per se* and are described, for example, in US 20150309016 A1.

For example, sandwich assays use a first antibody and a second antibody, and at least one of the first or second antibody used is preferably an antibody whose epitope is contained in an amino acid sequence represented by any one of SEQ ID NOs: 1 to 5, respectively. More preferably, the first antibody is at least one selected from the group consisting of an antibody whose epitope is contained in the amino acid sequence of SEQ ID NO: 1 and an antibody whose epitope is contained in the amino acid sequence of SEQ ID NO: 5, and is preferably a combination of both antibodies, while the second antibody is at least one selected from the group consisting of an antibody whose epitope is contained in the amino acid sequence of SEQ ID NO: 2, an antibody whose epitope is contained in the amino acid sequence of SEQ ID NO: 3, and an antibody whose epitope is contained in the amino acid sequence of SEQ ID NO: 4, and is preferably a combination of at least two, more preferably all three, of the antibodies.

Preferably, the first antibody is used as a capture antibody (an immobilized antibody) and the second antibody is used as a labeled antibody. Alternatively, the second antibody can be used as a capture antibody and the first antibody can be used as a labeled antibody.

Among the immunoassays described above, chemiluminescent enzyme immunoassay (CLEIA), chemiluminescent immunoassay (CLIA), enzyme immunoassay (EIA), radioimmunoassay (RIA), and fluorescence immunoassay (FIA) are immunoassays classified based on the types of labels used to perform a direct competitive assay, indirect competitive assay, or sandwich assay as described above. Chemiluminescent enzyme immunoassay (CLEIA) is an immunoassay that uses an enzyme (for example, alkaline phosphatase, as described above) as a label and a chemiluminescent compound-generating substrate (for example, AMPPD, as described above) as a substrate. Enzyme immunoassay (EIA) is an immunoassay that uses an enzyme (for example, peroxidase, alkaline phosphatase, luciferase, or β-galactosidase, as described above) as a label. The substrate for each of the enzymes is a compound that can be quantified, for example, by measuring absorbance. For example, when peroxidase is used, 1,2-phenylenediamine (OPD), 3,3'5,5'-tetramethylbenzidine (TMB), or the like is used; when alkaline phosphatase is used, p-nitrophenyl phosphate (pNPP) or the like is used; when β-galactosidase is used, 4-methylumbelliferyl galactoside (MG), nitrophenylgalactoside (NG), or the like is used; when luciferase is used, luciferin or the like is used. Radioimmunoassay (RIA) is a method that uses a radioactive material, and radioactive elements such as ³H, ¹⁴C, ³²P, ³⁵S, and ¹²⁵I are listed as radioactive materials as described above. Fluorescence immunoassay (FIA) is a method that uses a fluorescent substance or protein as a label, and fluorescein, fluorescein isothiocyanate, rhodamine, green fluorescent protein, red fluorescent protein, and the like are listed as fluorescent substances or proteins as described above. Immunoassays using these labels are well known *per se* and are described, for example, in US 8039223 B and US 20150309016 A1.

Turbidimetric immunoassay (TIA) is an immunoassay based on the phenomenon that the formation of an antigen-antibody complex by an antigen-antibody reaction between a target antigen to be measured (which in the present invention is Tg-SH) and an antibody directed against the antigen results in an increase in turbidity. The antigen is added at various known concentrations to a solution of the anti-target antigen antibody solution and the resulting turbidity is measured for each of the mixtures to generate a calibration curve. The amount of antigen in an unknown test sample can be determined by similarly measuring the turbidity of the unknown test sample and the interpolating the measured turbidity to the calibration curve. Turbidimetric immunoassay is well known *per se* and is described, for example, in US 20140186238 A1. Latex agglutination test is similar to turbidimetric immunoassay and is a method that uses a suspension of latex particles with an anti-target antigen antibody immobilized on the surface instead of an antibody solution in the turbidimetric immunoassay. Turbidimetric immunoassay and latex agglutination test are well known *per se* and are described, for example, in US7,820,398B.

Immunochromatography is a method in which a sandwich or competitive assay as described above is performed on a substrate (also called a matrix or strip) made of a porous material, such as filter paper, cellulose membrane, glass fiber, or nonwoven fabric. For example, in immunochromatography based on a sandwich assay, an anti-target antigen antibody is immobilized on a region of a substrate as described above to provide a detection zone, and a test sample containing a target antigen (which in the present invention is a sample obtained by the pretreatment step described above) is dropped onto the substrate, and a developer is applied from the upstream side to move the target antigen to the detection zone, thereby immobilizing the target antigen on the detection zone. The immobilized target antigen is sandwiched between the anti-target antigen antibody and a labeled secondary antibody to detect the label immobilized on the detection zone, resulting in the detection of the target antigen in the test sample. A labeling zone containing the labeled secondary antibody is provided upstream of the detection zone so that the complex of the target antigen and the labeled secondary antibody is immobilized on the detection zone. In cases where the label is an enzyme, a substrate zone containing the substrate of the enzyme is also provided upstream of the detection zone. For example, in immunochromatography based on a competitive assay, a target antigen is immobilized on a detection zone so that a target antigen in a test sample and the target antigen immobilized on the detection zone can compete with each other. The target antigen in the test sample can be detected or quantified by providing a labeled antibody zone upstream of the detection zone, reacting the target antigen in the test sample with a labeled antibody, and immobilizing any unreacted labeled antibody on the detection zone to detect or quantify the label. Immunochromatography is well known *per se* in the art and is described, for example, in US 6210898 B.

### <Assay kit for Tg-SH>

A kit of the present invention for Tg-SH measurement is a reagent with which the method of Tg-SH measurement described above can be conducted. The reagent for measurement of the present invention comprises at least a reducing agent and a monoclonal antibody whose corresponding antigen is Tg-SH.

The kit of the present invention may comprise the components in isolation from each other or in the form of a composition. In particular, the components may be provided stored in different containers (such as tubes or plates), or some of the components may be provided in the form of a composition (such as in a single solution). Alternatively, the kit of the present invention may be provided in the form of a device. In particular, all of the components may be provided stored in a device, or some of the components may be provided stored in a device with the remainder of the provided components not stored in the device (for example, stored in another container). In the latter case, the components not stored in the device may be injected into the device for use prior to measurement of a target substance.

In a preferred embodiment, the kit of the present invention may be appropriately composed depending on the type of immunoassay to be employed. For example, when a sandwich assay is employed, the kit of the present invention may comprise (i) a pretreatment solution, (ii) an antibody against Tg-SH, and (iii) a buffer as essential components and (iv) another antibody against Tg-SH, (v) a labeling substance, (vi) a diluent, and, if necessary, (vii) a substrate that reacts with the labeling substance as optional components. The components (ii) and (iii) may be contained in a single solution. The component (iv) may be labeled with the labeling substance (v). Preferably, the antibody against Tg-SH may be immobilized on magnetic particles.

The present invention will be hereinbelow specifically described by way of examples. However, the present invention is not limited to the examples described below.

### EXAMPLES

### 1. Production of Anti-Tg-SH Monoclonal Antibody

### (A) Preparation of Antigen for Immunization

Natural human thyroglobulin protein (abcam, ab96518) was prepared at 10 mg/mL using phosphate buffer (pH 7.2) containing 150 mM NaCl. A reducing agent, tris(2-carboxyethyl)phosphine (TCEP), was added to the solution to a final concentration of 10 mM, and the reaction was allowed to proceed at 37°C for 30 minutes. A desalting column was used for buffer exchange to phosphate buffer (pH 6.0) containing 1 mM EDTA and 150 mM NaCl to prepare reduced thyroglobulin (Tg-SH).

### (B) Immunization and Cell Fusion

The Tg-SH antigen protein prepared by the above method was diluted to a final concentration of 1.0 mg/ml with 10 mM phosphate-buffered saline (pH 7.3) (PBS) containing 150 mM NaCl, and the resulting solution was mixed with the same volume of Freund's adjuvant. The resulting mixture was administered subcutaneously to a BALB/c mouse at 4 to 6 weeks of age at a dose of 50-100 µg. The mouse was boosted every two weeks at the same dose and in the same manner, and 100 µg of the antigen protein dissolved in PBS was administered intraperitoneally to the mouse for the final immunization. Three days after the final immunization, the spleen was aseptically removed from the mouse to generate hybridomas by the conventional hybridoma technique, and the culture supernatants of the resulting hybridomas were collected and used for the screening described below.

### (C) Screening of Anti-Tg-SH Antibody-Producing Hybridoma

A hybridoma producing an antibody of interest was screened by ELISA using the above Tg-SH antigen protein. The Tg-SH antigen diluted to 1 µg/mL in TBS containing 1 mM TCEP and 6 M urea was added to a Nunc multimodule plate at 50 µL per well and allowed to stand overnight at 4-8°C. After removal of the diluted antigen solution, 100 µL of a blocking solution (0.1% casein and 1 mM EDTA in PBS) was added to each well and allowed to stand for 1 hour at room temperature to prepare an antigen-immobilized plate.

Each hybridoma culture supernatant was diluted with a reaction solution (0.1% casein and 1 mM EDTA in PBS) and added to the antigen-immobilized plate at 50 µL per well and allowed to stand for 1 hour at room temperature. The wells were washed with PBS containing 0.05% Tween 20 (trademark), and then 50 µL of horseradish peroxidase-labeled (HRP-labeled) anti-mouse IgG Fc-specific antibody was added to each well and allowed to stand for 1 hour at room temperature. After washing the wells with PBS containing 0.05% Tween 20 (trademark), 50 µL of TMB solution was added to each well to develop color. The reaction was then stopped by adding 2 M H₂SO₄ to each well, and the absorbance was measured at a wavelength of 450 nm to obtain hybridomas that produced an antibody with the desired reaction specificity.

### (D) Cloning of Hybridoma

The resulting hybridoma clones were subcloned by limiting dilution procedures to establish antibody-producing hybridoma lines. The resulting hybridomas were designated 9E12, A1010, A1024, A2029, and A2068.

### (E) Preparation of Anti-Tg-SH Monoclonal Antibody

Established antibody-producing hybridomas were cultured in a serum-free medium (Hybridoma-SFM, GIBCO) for adaptation. Each culture was expanded to 50 mL in a T75 flask, and was transferred to a culture bag (NIPRO) filled with 500 mL of a serum-free medium when the cell density reached approximately 5 × 10⁵ cells/ml. After culturing for 2 to 4 weeks, the supernatant was collected from each culture. The culture supernatant was applied to a column packed with Protein G Sepharose (GE Healthcare Technologies Inc.) and the bound antibody was eluted with pH 3 buffer. The eluted solution was neutralized with 2 M Tris (pH 8), and a desalting column was used for buffer exchange to PBS. Anti-Tg-SH monoclonal antibody of 5 to 20 mg was successfully obtained from 500 mL of each culture.

### 2. Identification of a Reaction Region in Anti-Tg-SH Antibody Using Partial-Length Thyroglobulin Antigen

The reaction region of each antibody was identified using six partial-length Tg antigens (TGN: 20-377 a.a., TG2F: 359-726 a.a., TG4F: 1074-1469 a.a., TG5F: 1470-1891 a.a., TG6F: 1892-2187 a.a., TGC: 2336-2768 a.a.; the amino acid sequences of the antigens are listed in Table 1) expressed in *E. coli.* The antigens, including Tg-SH used as an immunogen, were diluted to 1 µg/mL in TBS containing 1 mM TCEP and 6 M urea, added to a Nunc Multimodule Plate at 50 µL per well, and allowed to stand overnight at 4-8°C. After removal of each diluted antigen solution, 100 µL of a blocking solution (0.1% casein and 1 mM EDTA in PBS) was added to each well and allowed to stand for 1 hour at room temperature to prepare an antigen-immobilized plate.

Each anti-Tg-SH antibody was diluted in a reaction solution (0.1% casein and 1 mM EDTA in PBS) and added to the antigen-immobilized plate at 50 µL per well and allowed to stand for 1 hour at room temperature. The wells were washed with PBS containing 0.05% Tween 20 (trademark), and then 50 µL of horseradish peroxidase-labeled (HRP-labeled) anti-mouse IgG Fc-specific antibody was added to each well and allowed to stand for 1 hour at room temperature. After washing the wells with PBS containing 0.05% Tween 20 (trademark), 50 µL of TMB solution was added to each well to develop color. The reaction was then stopped by adding 2 M H₂SO₄ to each well, and the absorbance was measured at a wavelength of 450 nm. The result showed that 9E12 recognized TGN, and A1010 recognized TGC, and A1024, A2029 and A2068 recognized TG5F (Table 2).

**[Table 1]**

| Table 1 Amino acid sequences of partial-length thyroglobulin antigens | | |
|---|---|---|
| Name at partial-length antigen | Region (a.a) | Amino acid sequence |
| TGN | 20-377 | NIFEYQVDAQPLRPCELQRETAFLKQADYVPQCAEDGSFQTVQCQNDGRSCWCVGANGSE VLGSRQPGRPVACLSFCQLQKQQILLSGYINSTDTSYLPQCQDSGDYAPVQCDVQQVQCWC VDAEGMEVYGTRQLGRPKRCPRSCEIRNRRLLHGVGDKSPPQCSAEGEFMPVQCKFVNTTD MMIFDLVHSYNRFPDAFVTFSSFQRRFPEVSGYCHCADSQGRELAETGLELLLDEIYDTIFAGL DLPSTFTETTLYRILQRRFLAVQSVISGRFRCPTKCEVERFTATSFGHPYVPSCRRNGDYQAV QCQTEGPCWCVDAQGKEMHGTRQQGEPPSCAEGQSCASERQQALSRLYF |
| TG2F | 359-726 | AEGQSCASERQQALSRLYFGTSGYFSQHDLFSSPEKRWASPRVARFATSCPPTIKELFVDSGL LRPMVEGQSQQFSVSENLLKEAIRAIFPSRGLARLALQFTTNPKRLQQNLFGGKFLVNVGQFN LSGALGTRGTFNFSQFFQQLGLASFLNGGRQEDLAKPLSVGLDSNSSTGTPEAAKKDGTMNK PTVGSFGFEINLQENQNALKFLASLLELPEFLLFLQHAISVPEDVARDLGDVMETVLSSQTCEQ TPERLFVPSCTTEGSYEDVQCFSGECWCVNSWGKELPGSRVRGGQPRCPTDCEKQRARMQ SLMGSQPAGSTLFVPACTSEGHFLPVQCFNSECYCVDAEGQAIPGTRSAIGKPKKC |
| TG4F | 1074-1469 | PTTCEKSRTSGLLSSWKQARSQENPSPKDLFVPACLETGEYARLQASGAGTWCVDPASGEEL RPGSSSSAQCPSLCNVLKSGVLSRRVSPGYVPACRAEDGGFSPVQCDQAQGSCWCVMDSG EEVPGTRVTGGQPACESPRCPLPFNASEVVGGTILCETISGPTGSAMQQCQLLCRQGSWSVF PPGPLICSLESGRWESQLPQPRACQRPQLWQTIQTQGHFQLQLPPGKMCSADYADLLQTFQ VFILDELTARGFCQIQVKTFGTLVSIPVCNNSSVQVGCLTRERLGVNVTWKSRLEDIPVASLP DLHDIERALVGKDLLGRFTDLIQSGSFQLHLDSKTFPAETIRFLQGDHFGTSPRTWFGCSEGF YQVLTSEASQDGLGCVKCPEGSYSQ |
| TG5F | 1470-1891 | DEECIPCPVGFYQEQAGSLACVPCPVGRTTISAGAFSQTHCVTDCQRNEAGLQCDQNGQYR ASQKDRGSGKAFCVDGEGRRLPWWETEAPLEDSQCLMMQKFEKVPESKVIFDANAPVAVRS KVPDSEFPVMQCLTDCTEDEACSFFTVSTTEPEISCDFYAWTSDNVACMTSDQKRDALGNSK ATSFGSLRCQVKVRSHGQDSPAVYLKKGQGSTTTLQKRFEPTGFQNMLSGLYNPIVFSASGA NLTDAHLFCLLACDRDLCCDGFVLTQVQGGAIICGLLSSPSVLLCNVKDWMDPSEAWANAT CPGVTYDQESHQVILRLGDQEFIKSLTPLEGTQDTFTNFQQVYLWKDSDMGSRPESMGCRK DTVPRPASPTEAGLTTELFSPVDLNQVIVNGNQSLSSQKHWLFKHLFSAQQANLW |
| TG6F | 1892-2187 | CLSRCVQEHSFCQLAEITESASLYFTCTLYPEAQVCDDIMESNAQGCRLILPQMPKALFRKKVI LEDKVKNFYTRLPFQKLMGISIRNKVPMSEKSISNGFFECERRCDADPCCTGFGFLNVSQLKG GEVTCLTLNSLGIQMCSEENGGAWRILDCGSPDIEVHTYPFGWYQKPIAQNNAPSFCPLVVL PSLTEKVSLDSWQSLALSSVVVDPSIRHFDVAHVSTAATSNFSAVRDLCLSECSQHEACLITT LQTQPGAVRCMFYADTQSCTHSLQGQNCRLLLREEATHIYRKP |
| TGC | 2336-2768 | RVGVFGFLSSGSGEVSGNWGLLDQVAALTWVQTHIRGFGGDPRRVSLAADRGGADVASIHL LTARATNSQLFRRAVLMGGSALSPAAVISHERAQQQAIALAKEVSCPMSSSQEVVSCLRQKP ANVLNDAQTKLLAVSGPFHYWGPVIDGHFLREPPARALKRSLWVEVDLLIGSSQDDGLINRA KAVKQFEESRGRTSSKTAFYQALQNSLGGEDSDARVEAAATWYYSLEHSTDDYASFSRALEN ATRDYFIICPIIDMASAWAKRARGNVFMYHAPENYGHGSLELLADVQFALGLPFYPAYEGQFS LEEKSLSLKIMQYFSHFIRSGNPNYPYEFSRKVPTFATPWPDFVPRAGGENYKEFSELLPNRQ GLKKADCSFWSKYISSLKTSADGAKGGQSAESEEEELTAGSGLREDLLSLQEPGSKTYSK |

The partial sequences in Table 1 are represented by SEQ ID NOs: 6 to 11 in Sequence Listing.

**[Table 2]**

| Immobilized antigen | Sequence | 9E12 | A1010 | A1024 | A2029 | A2068 |
|---|---|---|---|---|---|---|
| Tg-SH | full length | 1.717 | 1.275 | 2.309 | 2.105 | 1.961 |
| Tg-N | 20-377 | 2.721 | 0.071 | 0.107 | 0.050 | 0.049 |
| Tg-2F | 359-726 | 0.038 | 0.142 | 0.604 | 0.218 | 0.197 |
| Tg-4F | 1074-1469 | 0.034 | 0.032 | 0.051 | 0.030 | 0.029 |
| Tg-5F | 1470-1891 | 0.152 | 0.129 | 2.617 | 2.659 | 2.427 |
| Tg-6F | 1892-2187 | 0.061 | 0.024 | 0.146 | 0.039 | 0.054 |
| Tg-C | 2336-2768 | 0.022 | 2.261 | 0.078 | 0.023 | 0.027 |

### 3. Analysis of Anti-Tg-SH Antibody Epitope

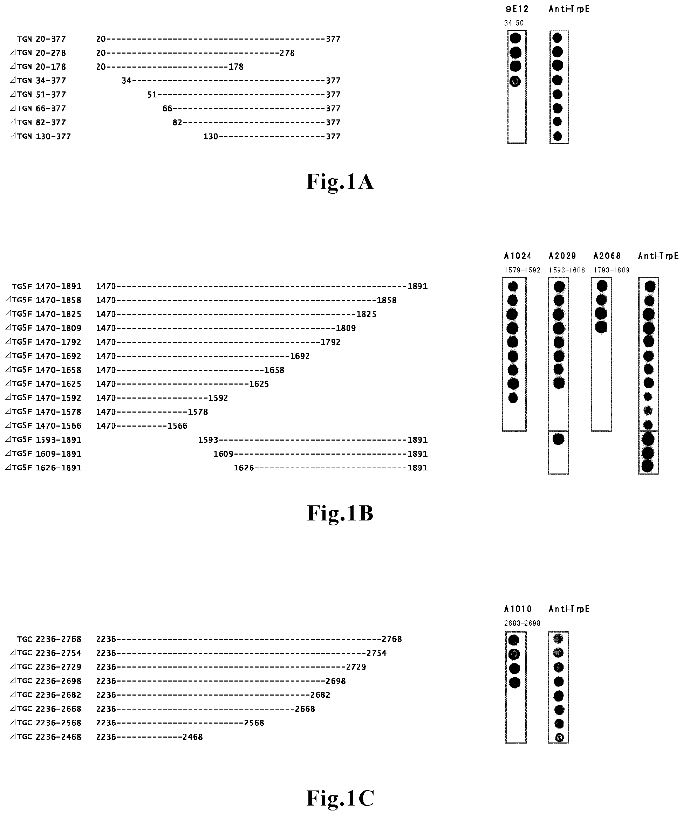

To analyze the epitopes of the obtained anti-Tg-SH antibodies, deletion mutant antigens of the above partial-length thyroglobulin antigens TGN, TG5F, and TGC were expressed in *E. coli*, and the results in Fig. 1A-C, which were obtained by the dot blot analysis using the homogenates of the resulting bacteria, showed that the epitope of the 9E12 antibody was contained in the sequence within 34-50 a.a., CELQRETAFLKQADYVP (SEQ ID NO: 1), and the epitope of the A1024 antibody was contained in the sequence within 1579-1592 a.a., VIFDANAPVAVRSK (SEQ ID NO: 2), and the epitope of the A2029 antibody was contained in the sequence within 1593-1608 a.a., VPDSEFPVMQCLTDCT (SEQ ID NO: 3), and the epitope of the A2068 antibody was contained in the sequence within 1793-1809 a.a., LGDQEFIKSLTPLEGTQ (SEQ ID NO: 4), and the epitope of the A1010 antibody was contained in the sequence within 2683-2698 a.a., TPWPDFVPRAGGENYK (SEQ ID NO: 5). The numerical values in Fig. 1A-C (for example, "20-377" in TGN20-377) represent the positions of amino acids in the full-length thyroglobulin protein (SEQ ID NO: 12). The anti-TrpE antibody was used to confirm that the proteins of interest were immobilized on the nitrocellulose membranes used in this analysis.

### 4. Evaluation of Anti-Tg-SH Monoclonal Antibody Reactivity

The reactivities of the obtained Tg-SH monoclonal antibodies were compared between unreduced Tg and reduced Tg (Tg-SH). Natural human thyroglobulin protein (abcam, ab96518) was diluted to 1 µg/mL with PBS to prepare unreduced Tg and with PBS containing 5 mM TCEP to prepare Tg-SH, and these antigens were added to a Nunc multimodule plate at 50 µL per well and allowed to stand overnight at 4-8°C. After removal of each diluted antigen solution, 100 µL of a blocking solution (0.1% casein and 1 mM EDTA in PBS) was added to each well and allowed to stand for 1 hour at room temperature to prepare an antigen-immobilized plate. Each anti-Tg antibody or each anti-Tg-SH antibody was diluted in a reaction solution (0.1% casein and 1 mM EDTA in PBS) and added to the antigen-immobilized plate at 50 µL per well and allowed to stand for 1 hour at room temperature. The wells were washed with PBS containing 0.05% Tween 20 (trademark), and then 50 µL of alkaline phosphatase-labeled (ALP-labeled) anti-mouse IgG Fc-specific antibody was added to each well and allowed to stand for 1 hour at room temperature. After washing the wells with PBS containing 0.05% Tween 20 (trademark), 50 µL of substrate solution containing AMPPD (Lumipulse (registered trademark) substrate solution, manufactured by Fujirebio Inc.) was added to each well for measurement of chemiluminescence generated by an enzymatic reaction on a plate reader.

The results are shown in Table 3. Conventional anti-Tg antibodies A and B showed reactivity to Tg-SH that was not more than 10% of the reactivity to unreduced Tg, whereas the obtained anti-Tg-SH antibodies showed higher reactivity to Tg-SH than to unreduced Tg.

**[Table 3]**

| Table 3 Comparison of antibody reactivities between unreduced Tg and Tg-SH | | | | |
|---|---|---|---|---|
| | | Unreduced Tg | Tg-SH | versus Unreduced |
| Anti-Tg antibodies | A | 75430 | 7090 | 7.7 |
| | B | 287830 | 14770 | 4.8 |
| Anti-Tg-SH antibodies | 9E12 | 13510 | 16890 | 126.5 |
| | A1010 | 4380 | 4780 | 119.0 |
| | A1024 | 4520 | 15550 | 504.2 |
| | A2029 | 55570 | 77340 | 139.9 |
| | A2068 | 10180 | 23150 | 244.0 |

### 5. Construction of Highly Sensitive Automatic Analytical Measuring System

A highly sensitive automatic analytical measuring system was constructed below by (A) preparing antibody-conjugated ferrite particles and (B) preparing an alkaline phosphatase-labeled (ALP-labeled) antibody.

### (A) Preparation of Antibody-Conjugated Magnetic Particle

*N*-hydroxysuccinimide (NHS) and 1-ethyl-3-(3-dimethylaminopropyl)carbodiimide hydrochloride (EDC hydrochloride) were mixed with magnetic particles, and the reaction was allowed to proceed for 30 minutes at room temperature. After washing the ferrite particles, an anti-Tg-SH antibody or an anti-Tg antibody was added to the ferrite particles, and the mixture was stirred with an end-over mixer for 60 minutes at room temperature. The reaction was stopped by adding 1 M Tris (pH 7.0) to the mixture and stirring the resulting mixture with an end-over mixer for 30 minutes at room temperature. The particles were suspended in storage buffer (50 mM Tris, 2.0% BSA, 150 mM NaCl, 1 mM EDTA, 0.1% NaN₃, pH 7.2) to prepare antibody-conjugated magnetic particles.

### (B) Preparation of Alkaline Phosphatase-Labeled (ALP-Labeled) Antibody

A desalted monoclonal antibody was digested with pepsin, and the resulting digest was purified by gel filtration using coupling buffer (0.1 M phosphate buffer, 1 mM EDTA, pH 6.0) to obtain an antibody without the Fc region. The antibody was then mixed with 2-mercaptoethylamine (final concentration: 10 mM) and the resulting mixture was allowed to stand at 37°C for 3 hours for thiolation. The resulting antibody was further desalted with coupling buffer to yield a Fab' antibody. Meanwhile, a desalted alkaline phosphatase and N-(4-maleimidobutyryloxy)-succinimide (GMBS) were mixed in 0.1 M phosphate buffer (pH 7.0), and the resulting mixture was allowed to stand at 30°C for 1 hour for maleimidation. The maleimidated alkaline phosphatase was desalted with coupling buffer and then mixed with the Fab' at a molar ratio of 1:1, and the resulting mixture was allowed to stand at 25°C for 30 minutes for coupling. The reaction was stopped by adding 2-mercaptoethylamine to the coupling solution (final concentration: 2 mM) and allowing the resulting mixture to stand for 30 minutes at room temperature. Iodoacetamide was further added to the mixture (final concentration: 10 mM) and the resulting mixture was allowed to stand for 30 minutes at room temperature to block free thiol groups. The mixture was concentrated and purified by gel filtration to obtain an ALP-labeled antibody.

### (C) Antibody Combination

An unreduced Tg measurement system (hereinafter referred to as Tg measurement system) was constructed by combining the anti-Tg antibody A-conjugated ferrite particles and the ALP-labeled anti-Tg antibody B, while a Tg-SH measurement system was constructed by combining ferrite particles co-conjugated with 9E12 and A1010 as capture antibodies and a mixture of A1024, A2029, and A2068 as ALP-labeling materials (labeled antibodies).

### 6. Test for Confirming Effect of Reducing Pretreatment

### (A) Preparation of Reducing Pretreatment Solution

A pretreatment solution had a basal composition of 2 M arginine hydrochloride and 50 mM Tris at pH 7.5, and 0 - 500 mM 2-DEAET (2-(diethylamino)ethanethiol hydrochloride), 0 - 50 mM DTT (dithiothreitol), or 0 - 50 mM TCEP (tris(2-carboxyethyl)phosphine hydrochloride) as a reducing agent was added to the pretreatment solution to prepare reducing pretreatment agents.

### (B) Preparation of TgAb Positive Model Sample

Natural human thyroglobulin protein (abcam, ab96518) was diluted with horse serum, and the TgAb calibrator (3000 IU/mL) from Lumipulse Presto TgAb (Fujirebio Inc.) was added to the dilution to give a concentration of 500 IU/mL to prepare a TgAb positive model sample [TgAb(+)]. On the other hand, the TgAb calibrator (0 IU/mL) was added in the same manner to prepare a TgAb negative model sample [TgAb(-)].

### (C) Measurement Method

A 30-µL aliquot of a sample was mixed with a 90-µL aliquot of a pretreatment solution and the resulting mixture was allowed to stand for 30 minutes at room temperature to prepare a pretreated sample. Fifty µL of the pretreated sample was mixed with 50 µL of a magnetic particle suspension (50 mM Tris-HCl, 150 mM NaCl, 20 mM EDTA 3Na, 5% cold-water fish skin gelatin, 1 M arginine hydrochloride, 100 mM iodoacetamide, pH 7.5) containing anti-Tg-SH antibody-conjugated or anti-Tg antibody-conjugated magnetic particles, and the reaction was allowed to proceed at 37°C for 8 minutes. The magnetic particles were collected with magnets, washed to remove components not bound to the magnetic particles, mixed with 50 µL of ALP-labeled anti-Tg-SH antibody or the ALP-labeled anti-Tg antibody diluted in label diluent (50 mM MES, 100 mM NaCl, 0.3 mM ZnCl₂, 1 mM MgCl₂, 2% BSA, 5 µg/mL inactive ALP, 0.1% ProClin300, pH 6.8) to 0.2 µg/mL, and allowed to react at 37°C for 8 minutes. The magnetic particles were collected with magnets, washed to remove components not bound to the magnetic particles, and mixed with 200 µL of a substrate solution containing AMPPD (Lumipulse (registered trademark) substrate solution, manufactured by Fujirebio Inc.) to measure the intensity of the luminescence generated by the enzymatic reaction. The steps following the sample pretreatment step in this example were all performed using a Lumipulse (registered trademark) L2400 automated analyzer (manufactured by Fujirebio Inc.).

### (A) Results

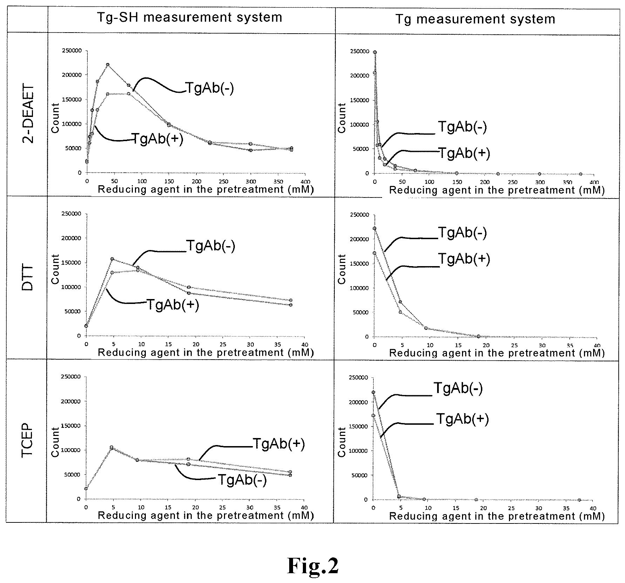

Fig. 2 shows the results of pretreatment with different types of reducing agents. In the Tg measurement system, the signal of the TgAb(+) sample was lower than that of the TgAb(-) sample when no reducing agent was added, indicating the interference of Tg measurement with TgAb. The influence of TgAb is decreased as the reducing agent concentration increases. However, the signal is also significantly reduced, making Tg measurement difficult at a reducing agent concentration where the signal values of TgAb(+) and TgAb(-) are completely identical (where TgAb is completely free). On the other hand, in the Tg-SH measurement system, the reactivity was significantly increased by the addition of a reducing agent, indicating that Tg measurement is possible at a reducing agent concentration where TgAb is completely free. Tg can be measured without interference from TgAb when the concentration of 2-DEAET is 150 mM or more, when the concentration of DTT is 10 mM or more, and when the concentration of TCEP is 5 mM or more, in the pretreatment.

### 7. Test Using Human Sample for Confirming Effect of Reducing Pretreatment

### (A) Preparation of Reducing Pretreatment Solution

The composition of a pretreatment solution was investigated with the goal of preventing the aggregation of proteins in a sample or the increase in viscosity of a sample by reduction treatment, thereby improving handling efficiency and measurement sensitivity, and was found to be 2.4 M urea, 6.4% C16APS *(N-*hexadodecyl-*N,N*-dimethyl-3-ammonio-1-propanesulfonate), 0.16MNaCl, 50 mM Tris, and 20 mM TCEP at pH 7.5. A pretreatment solution having the same composition as above, but without TCEP, was used in a control Tg measurement system.

### (B) Preparation of TgAb Positive Model Sample

To prepare model samples, a sample diluent (Fujirebio Inc.) or a high Tg sample was added to thyroid-related disease or healthy sera in which the TgAb content had been measured using the TgAbs from Lumipulse Presto TgAb (Fujirebio Inc.). Additionally, a dilution series of natural human thyroglobulin protein (nhTg, abcam, ab96518) was prepared using Lumipulse Sample Diluent (Fujirebio Inc.) and another dilution series of nhTg was prepared by adding the TgAb calibrator from Lumipulse Presto TgAb (Fujirebio Inc.) to give a concentration of 750 IU/mL. Samples #1-26 listed in Table 4 were prepared in this manner.

### (C) Measurement Method

A 30-µL aliquot of a sample was mixed with a 90-µL aliquot of a pretreatment solution and the resulting mixture was allowed to stand for 30 minutes at room temperature to prepare a pretreated sample. Fifty µL of the pretreated sample was mixed with 50 µL of a magnetic particle suspension (100 mM Tris-HCl, 200 mM NaCl, 20 mM EDTA 3Na, 0.1% ProClin 300, 2% BSA, pH 7.5) containing anti-Tg-SH antibody-conjugated or anti-Tg antibody-conjugated magnetic particles, and the reaction was allowed to proceed for 8 minutes. The magnetic particles were collected with magnets, washed to remove components not bound to the magnetic particles, mixed with 50 µL of ALP-labeled anti-Tg-SH antibody or the ALP-labeled anti-Tg antibody diluted in label diluent (50 mM MES, 0.1 mM ZnCh, 1 mM MgCh, 3% BSA, 5 µg/mL inactive ALP, 0.10% NaN₃, pH 6.8) to 0.2 µg/mL, and allowed to react for 8 minutes. The magnetic particles were collected with magnets, washed to remove components not bound to the magnetic particles, and mixed with 200 µL of a substrate solution containing AMPPD (Lumipulse^{®} Substrate Solution, manufactured by Fujirebio Inc.). The intensity of the luminescence generated by the enzymatic reaction was measured, and the Tg value in the sample was calculated from the intensity count using a calibration curve. The calibration curve was generated from the luminescence intensities measured for standard solutions of natural human thyroglobulin protein (nhTg, abcam, ab96518) containing 0, 10, 50, 200, or 1000 ng/mL Tg content in the same manner as the samples. The steps following the sample pretreatment step in this example were all performed using a Lumipulse^{®} L2400 automated analyzer (manufactured by Fujirebio Inc.).

### (A) Results

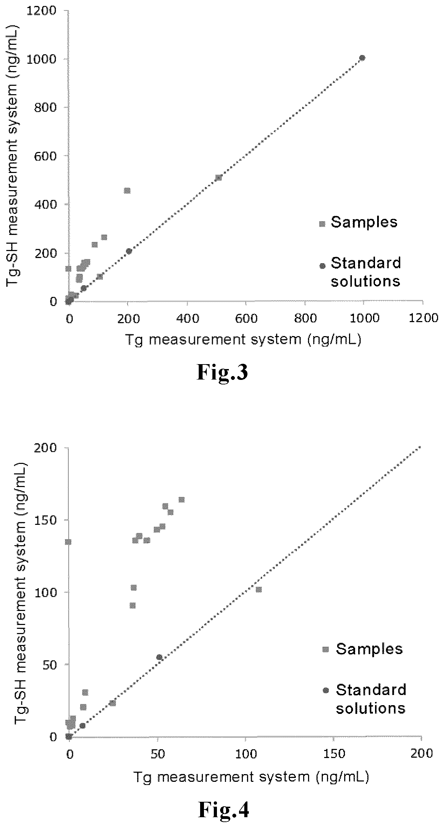

Table 4 shows the results of Tg-SH and Tg measurements. Fig. 3 shows the correlation between the Tg-SH and Tg measurements, and Fig. 4 is a magnified view of Fig. 3 in the low concentration range. The Tg value measured by the Tg-SH measurement system was often found to be twice or higher than that measured by the Tg measurement system. In particular, Tg in samples #7 and #13 was not detected by the Tg measurement system, but was successfully detected by the Tg-SH measurement system. The above results have demonstrated that the Tg-SH measurement of the present invention allows the avoidance of measurement interference by TgAb and alleviates false low readings in Tg measurement and diagnosis.

**[Table 4]**

| **Table 4 Comparison between Tg-SH and Tg measurements** | | | | | | | |
|---|---|---|---|---|---|---|---|
| Sample | | | | TgAb | Tg-SH measurement system Tg (ng/mL) | Tg measurement system Tg (ng/mL) | Tg-SH/Tg |
| | | | | (IU/mL) | | | |
| #1 | Thyroid-related disease test sample | | + sample diluent | 25 | 9.1 | 2.2 | 4.2 |
| #2 | Thyroid-related disease test sample | | + sample diluent | 31 | 0.0 | 0.0 | - |
| #3 | Thyroid-related disease test sample | | + sample diluent | 2569 | 0.0 | 0.0 | |
| #4 | Thyroid-related disease test sample | | + sample diluent | 98 | 12.4 | 2.7 | 4.7 |
| #5 | Thyroid-related disease test sample | | + sample diluent | 32 | 142.8 | 50.1 | 2.9 |
| #6 | Thyroid-related disease test sample | | + sample diluent | 51 | 103.0 | 37.4 | 2.8 |
| #7 | Thyroid-related disease test sample | | + sample diluent | 25 | 9.6 | 0.0 | |
| #8 | Healthy test sample | | + sample diluent | 17 | 7.1 | 1.9 | 3.7 |
| #9 | Healthy test sample | | + sample diluent | 34 | 6.8 | 0.4 | 15.2 |
| #10 | Healthy test sample | | + sample diluent | 22 | 30.4 | 9.4 | 3.2 |
| #11 | Thyroid-related disease test sample | | + high Tg | 25 | 144.6 | 53.3 | 2.7 |
| #12 | Thyroid-related disease test sample | | + high Tg sample | 31 | 135.2 | 38.0 | 3.6 |
| #13 | Thyroid-related disease test samole | | + high Tg sample | 2569 | 134.2 | 0.0 | |
| #14 | Thyroid-related disease test sample | | + high Tg sample | 98 | 135.2 | 44.6 | 3.0 |
| #15 | Thyroid-related disease test sample | | + high Tg sample | 32 | 264.5 | 120.4 | 2.2 |
| #16 | Thyroid-related disease test sample | | + high Tg sample | 51 | 234.5 | 89.2 | 2.6 |
| #17 | Thyroid-related disease test sample | | + high Tg sample | 25 | 138.4 | 40.5 | 3.4 |
| #18 | Healthy test sample | | + high Tg sample | 17 | 158.8 | 55.2 | 2.9 |
| #19 | Healthy test sample | | + high Tg sample | 34 | 154.6 | 58.2 | 2.7 |
| #20 | Healthy test sample | | + high Tg sample | 22 | 163.5 | 64.4 | 2.5 |
| #21 | nhTg | 500 ng/mL | + sample diluent | 0 | 510.0 | 511.0 | 1.0 |
| #22 | nhTg | 100 ng/mL | + sample diluent | 0 | 101.1 | 108.3 | 0.9 |
| #23 | nhTg | 25 ng/mL | + sample diluent | 0 | 22.9 | 25.2 | 0.9 |
| #24 | nhTg | 500 ng/mL | +TgAb | 750 | 456.6 | 201.1 | 2.3 |
| #25 | nhTg | 100 ng/mL | +TgAb | 750 | 90.4 | 36.5 | 2.5 |
| #26 | nhTg | 25 ng/mL | +TgAb | 750 | 20.2 | 8.2 | 2.5 |

## Claims

1. A method of measuring thyroglobulin by immunoassay in a sample isolated from the body, comprising a pretreatment step of treating the sample isolated from the body with a reducing agent, wherein a monoclonal antibody or an antigen-binding fragment thereof whose corresponding antigen is thyroglobulin treated with the reducing agent is used for the immunoassay.

2. The method of claim 1, wherein the corresponding epitope of the monoclonal antibody is contained in an amino acid sequence of any one of SEQ ID NOs: 1 to 5.

3. The method of claim 1 or 2, wherein the immunoassay is a sandwich assay and wherein the monoclonal antibody or an antigen-binding fragment thereof is used as at least one of a first antibody or a second antibody in the sandwich assay.

4. The method of claim 3, wherein the first antibody comprises at least one selected from the group consisting of an antibody whose corresponding epitope is contained in the amino acid sequence of SEQ ID NO: 1 and an antibody whose corresponding epitope is contained in the amino acid sequence of SEQ ID NO: 5, and
the second antibody comprises at least one selected from the group consisting of an antibody whose corresponding epitope is contained in the amino acid sequence of SEQ ID NO: 2, an antibody whose corresponding epitope is contained in the amino acid sequence of SEQ ID NO: 3, and an antibody whose corresponding epitope is contained in the amino acid sequence of SEQ ID NO: 4.

5. The method of claim 4, wherein the first antibody is a capture antibody and the second antibody is a labeled antibody.

6. An immunoassay kit for use in the method of claim 1, comprising a reducing agent and a monoclonal antibody or an antigen-binding fragment thereof whose corresponding antigen is thyroglobulin treated with the reducing agent.
